# EUROPÄISCHE PATENTSCHRIFT

(11) **EP 2 844 616 B1**
(45) Veröffentlichungstag und Bekanntmachung des Hinweises auf die Patenterteilung: **24.02.2016**
(21) Anmeldenummer: 13717426.4
(22) Anmeldetag: 06.03.2013
(51) Int. Cl.: C02F 1/32, B01J 19/12

(54) **UV-WASSERBEHANDLUNGSANLAGE MIT OFFENEM KANAL**
OPEN-CHANNEL UV WATER TREATMENT PLANT
INSTALLATION DE TRAITEMENT DE L'EAU PAR UV PRÉSENTANT UN CONDUIT OUVERT

(30) Priorität: 04.05.2012 DE 102012008733
(43) Veröffentlichungstag der Anmeldung: 11.03.2015
(73) Patentinhaber: Xylem Water Solutions Herford GmbH, 32051 Herford (DE)
(72) Erfinder: MORNINGSTAR, Leroy, Jack, Jr., Charlotte, NC 28273 (US); KÄMMERER, Sven, 32107 Bad Salzuflen (DE); RAPAKA, Madhukar, Warangal 506001 Andhra Pradesh (IN); KRÜGER, Friedhelm, 32657 Lemgo (DE); RAYMOND, Daniel, York, SC 29745 (US)
(74) Vertreter: Lenzing Gerber Stute
(86) Internationale Anmeldenummer: PCT/EP2013/000655
(87) Internationale Veröffentlichungsnummer: WO 2013/164049

(56) Entgegenhaltungen:
- WO-A1-99/14161
- AU-A4- 2010 100 165
- DE-A1- 4 119 725
- US-A- 5 564 765
- US-A1- 2004 069 954
- US-A1- 2008 260 602

## Beschreibung

Die vorliegende Erfindung betrifft eine UV-Wasserbehandlungsanlage mit den Merkmalen des Oberbegriffs des Anspruchs 1.

Es ist seit Langem bekannt, dass UV-Strahlung keimtötend wirkt und das bereits die natürlich auftretende UV-Strahlung im Sonnenlicht bei ausreichender Intensität und Dauer eine desinfizierende Wirkung hat. Für die Desinfektion von Wasser und Abwasser wird UV-Strahlung in kleinen und großen Anlagen eingesetzt, siehe z.B. die Patentschrift US 1,150,117. Dabei kann eine Unterscheidung getroffen werden zwischen Anlagen, bei denen UV-Strahler in geschlossenen Kanälen angeordnet sind und solchen Anlagen, bei denen die UV-Strahler in oben offenen Kanälen, sogenannten Gerinnen, angeordnet sind. Die zweite Bauart mit offenem Gerinne wir vorwiegend in der Abwassertechnik eingesetzt. Das geklärte Abwasser wird dabei durch einen offenen Kanal zu der UV-Anlage geleitet und dort mit UV-Strahlung beaufschlagt, um die Keimzahl so weit zu reduzieren, dass das geklärte Abwasser beispielsweise in Gewässer eingeleitet werden kann. Die Desinfektionsleistung kann dabei so hoch sein, dass die Einleitung in Badegewässer zulässig ist.

Abwasserbehandlungsanlagen sind normalerweise so aufgebaut, dass das Wasser allein auf Grund der Schwerkraft von einem Zulauf durch die verschiedenen Behandlungsstufen zu einem Auslauf strömt, ohne dass Pumpen erforderlich sind. Deshalb wird auch bei UV-Behandlungsanlagen in der Abwassertechnik angestrebt, den Strömungswiderstand möglichst gering zu halten, um bei der vorgesehenen Durchflussrate einen ebenfalls geringen Druckverlust zu erzielen. Ein solcher Druckverlust wird sich im Betrieb der Anlage in einer Höhendifferenz zwischen dem Wasserspiegel im Zulauf und dem Wasserspiegel im Ablauf ausdrücken. Man ist bestrebt, diese Höhendifferenz möglichst gering zu halten.

Wegen des besonders günstigen elektrischen Wirkungsgrads werden bei der Wasserdesinfektion vorwiegend so genannte Quecksilberniederdruckstrahler eingesetzt, die ein langgestrecktes Lampenrohr aus Quarz aufweisen, in dem eine Gasentladung zwischen zwei Elektroden die UV-Strahlung erzeugt. Die Länge eines solchen Strahlers beträgt ca. 1,5 m. Die Strahler werden zum Schutz vor dem Wasser und zur Aufrechterhaltung der Betriebstemperatur wasserdicht in Hüllrohre aus Quarz eingesetzt, die dann wiederum in dem zu desinfizierenden Wasser angeordnet sind. Diese Baugruppe aus UV-Strahler und Hüllrohr wird nachfolgend als Strahler bezeichnet.

Kommerzielle Anlagen, wie sie zum Beispiel im Ablauf von kommunalen Kläranlagen eingesetzt werden, haben normalerweise eine Vielzahl von Strahlern, teilweise über 100 Stück.

Bei Anlagen mit offenem Kanal, auch "Gerinne" genannt, gibt es verschiedene Anordnungen der Strahler. Die Strahler können horizontal und parallel zu der Strömungsrichtung in dem Kanal liegen (siehe z.B. die Patentschriften US 4,482,809 und US 6,500,312). Die Strahler können auch horizontal und quer zu der Strömungsrichtung liegen, siehe die Patentschrift US 4,367,410. Schließlich gibt es Anlagen, bei denen die Strahler senkrecht in dem Kanal stehen oder hängen, z.B. in den Patentschriften US 5,660,719 und US 5,332,388.

Mehrere UV-Strahler sind in Modulen zusammengefasst. Diese Module müssen zu Wartungszwecken aus dem Kanal gehoben werden, um beispielsweise einzelne Lampen auszutauschen oder die Strahleroberfläche zu reinigen. Kleinere Module mit etwa vier bis acht Strahlern können manuell aus dem Kanal herausgehoben werden. Größere Module erfordern den Einsatz eines Krans. Alternativ können Module auch an einem Ende (stromabwärts oder stromaufwärts) schwenkbar gelagert sein, wie dies beispielsweise in der US-Patentanmeldung US 2008/0260602 A1 gezeigt ist. Ein Modul kann dann aus dem Kanal nach oben herausgeschwenkt werden. Bei der genannten Patentanmeldung ist hierzu ein Handgriff vorgesehen, der das manuelle Verschwenken des Moduls ermöglicht. Bei größeren Modulen ist auch bekannt, einen Schwenkantrieb vorzusehen, der das Modul nach oben aus dem Kanal herausschwenkt.

Insbesondere für größere Module, die beispielsweise mehr als zwanzig Strahler aufweisen können, ist das Herausheben mit einem Kran nachteilig, weil zum einen eine schwere hängende Last bewegt werden muss und zum anderen bei senkrecht montierten Strahlern eine offene Stelle in dem Kanal entsteht, die sehr tief ist und entsprechend eine Absturzgefahr für Personal darstellt. Beide Umstände stellen ein potentielles Unfallrisiko dar. Bei Modulen, die mit einem Schwenkantrieb versehen sind, ist durch die Schwenkbewegung in Strömungsrichtung ein freier Platz im Kanal erforderlich, der der Gesamtlänge des Moduls entspricht. Hierfür ist es wiederum erforderlich, den Kanal auf dieser Länge freizuhalten. Es entsteht eine entsprechend lange Zone, in der eine Absturzgefahr für das Personal entstehen kann. Die nach oben geschwenkten Module befinden sich im herausgeschwenkten Zustand oberhalb des Kanals, der entsprechend abgedeckt werden muss, um die einzelnen Strahler warten zu können. Dies wird in der Praxis auch als Nachteil empfunden.

Es ist deshalb Aufgabe der vorliegenden Erfindung, eine UV-Wasserbehandlungsanlage mit Modulen, die mehrere UV-Strahler enthalten, dahingehend zu verbessern, dass die Wartung der Module und der Strahler außerhalb des Kanals einfacher und sicherer wird.

Diese Aufgabe wird von einer UV-Wasserbehandlungsanlage mit den Merkmalen des Anspruchs 1 gelöst.

Weil die Module mit einer Linearführung versehen sind, die im Wesentlichen parallel zu der Längsachse der UV-Strahler verläuft, und weil ein Antriebsmittel vorgesehen ist, das die Module gegenüber einem an den Kanal befestigten Führungselement verfahren kann, ist es möglich, die Module in Richtung der Längsachse der Strahler aus dem Kanal herauszufahren, sodass kein zusätzlicher offener Bereich des Kanals erforderlich ist. Die beim Herausfahren des Moduls erforderliche freie Oberseite des Kanals ist dadurch minimal. Es ist vorteilhaft, wenn das Modul eine Bodenplatte aufweist, die im herausgefahrenen Zustand des Moduls die Oberseite des Kanals schließt.

Der Sockel kann beidseitig an den Seitenwandungen des Kanals befestigt werden. Vorzugsweise erfolgt dies oberhalb der Wasserlinie des Kanals, sodass der Sockel und vorzugsweise auch das Antriebsmittel nicht dauerhaft mit dem Wasser in Kontakt kommen. Der Sockel weist vorzugsweise wenigstens zwei Führungen auf, die seitlich in der Nähe der Kanalwandung angeordnet sind. In diesen Führungen können Schienen geführt werden, die an dem Modul befestigt sind und die eine lineare Verschiebung des Moduls gegenüber dem Sockel ermöglichen. Das Antriebsmittel ist vorzugsweise ein Elektromotor, der über einen Ritzel eine mit dem Modul verbundene Zahnstange antreibt. Hierbei wird eine besonders hohe Betriebssicherheit erzielt, wenn der Elektromotor über ein selbsthemmendes Getriebe mit dem Ritzel antriebsmäßig verbunden ist. So kann auch bei einem plötzlichen Stromausfall verhindert werden, dass das angehobene Modul unkontrolliert in den Kanal abgesenkt wird. Alternativ kann auch ein elektromotorisch betriebener Seilzug, ebenfalls mit vorzugsweise selbsthemmendem Getriebe vorgesehen sein. Es können auch hydraulische oder pneumatische Antriebe angesetzt werden, die mit Kolben/Zylinderaggregaten wirken.

Besonders vorteilhaft ist es, ein Modul mit Linearführung und einem Antrieb zum Herausheben des Moduls aus dem Kanal so anzuordnen, dass die Strahler mit ihrer Längsachse parallel zu der Ebene der benachbarten Seitenwand des Kanals ausgerichtet sind und mit ihrer Längsachse in einem Winkel gegen die Strömungsrichtung geneigt sind. Der Winkel beträgt vorzugsweise zwischen dreißig und achtzig Grad besonders bevorzugt wird ein Bereicht zwischen vierzig und sechzig Grad zwischen der Strahlerlängsachse und der im Wesentlichen horizontalen Strömungsrichtung. Bei einem solchen Winkel ergibt sich ein besonders einfacher Zugang zu den Strahlern, wenn das Modul aus dem Kanal herausgehoben ist.

Nachfolgend wird ein Ausführungsbeispiel der vorliegenden Erfindung anhand der Zeichnung beschrieben. Es zeigen:
- Figur 1:: ein Modul mit einem Sockel und einem Linearantrieb in einer perspektivischen Ansicht von oben;
- Figur 2:: das Modul aus Figur 1 ohne den Sockel in einer anderen perspektivischen Darstellung;
- Figur 3:: den mechanischen Aufbau des Moduls aus Figur 2 ohne Abdeckungen der elektrischen Teile und ohne Strahler;
- Figur 4:: einen Sockel für das Modul gemäß Figur 1 in einer perspektivischen Darstellung ohne Antriebsmittel; sowie
- Figur 5:: den Sockel aus Figur 4 mit einem elektromotorischen Antrieb.

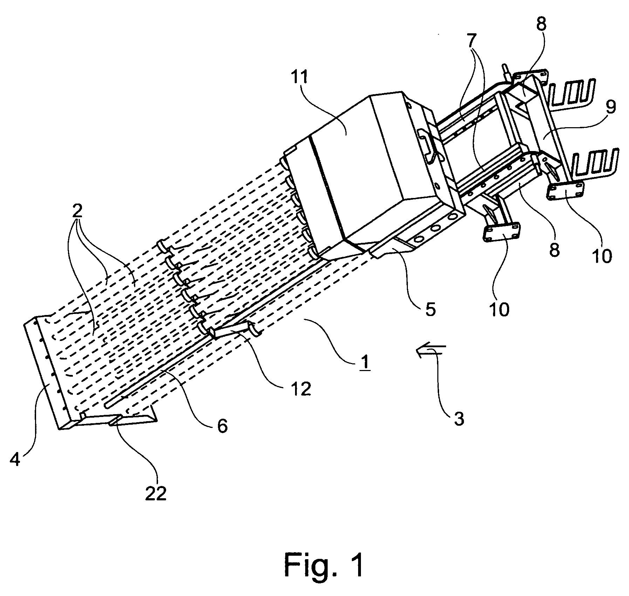

In der Figur 1 ist perspektivisch ein insgesamt mit 1 bezeichnetes Modul einer UV-Wasserbehandlungsanlage dargestellt. Das Modul umfasst insgesamt zwölf Strahler 2, die jeweils einen eher nicht näher dargestellten, langgestreckten UV-Quecksilberniederdruckstrahler und ein den Strahler umgebendes Hüllrohr aus UV-durchlässigem Material umfassen. Das Modul 1 ist zum Einbau in einen Kanal vorgesehen, in dem Wasser in Richtung der mit einem Pfeil angedeuteten Strömungsrichtung 3 schließt. Das Modul weist eine Bodenplatte 4 auf, die mit ihrer in Figur 1 nicht sichtbaren Unterseite auf dem Boden des Kanals aufsteht. Weiter weist das Modul 1 einen oberen Rahmen 5 auf, sowie Längsstreben 6, die die Bodenplatten 4 mit dem Rahmen 5 verbinden. An der in Figur 1 rückwärtigen Seite des Moduls, die der Strömungsrichtung 3 zugewandt ist, sind weiter zwei Führungsschienen 7 angebracht, die ebenfalls die Bodenplatte 4 und den Rahmen 5 verbinden und die im Wesentlichen parallel zu den langgestreckten Strahlern 2 und den Streben 6 verlaufen. Die Führungsschienen 7 stehen über den Rahmen 5 hinaus. Die Führungsschienen 7 stehen weiter im Eingriff mit einem Sockel 8, der mit Querstreben 9 und Befestigungsflanschen 10 an den Seitenwänden des hier nicht dargestellten Kanals oberhalb der Wasserlinie befestigbar ist. Der Sockel 8 weist in der Figur 1 nicht erkennbare Führungen auf, in denen die Führungsschienen 7 in Richtung ihrer Längserstreckung verschieblich gelagert sind.

Weiter ist das Modul 1 im Bereich des Rahmens 5 mit einer Abdeckhaube 11 versehen, die die elektrischen Anschlüsse der Strahler 2 nach oben oberhalb der Wasserlinie in den Kanal abdeckt. Schließlich trägt das Modul 1 noch eine Reinigungsvorrichtung 12, die einen Halter und je einen Reinigungsring für jeden Strahler 2 umfasst. Die Reinigungseinrichtung 12 ist, wie aus dem Stand der Technik bekannt, in Längsrichtung der Strahler 2 verschieblich, sodass bei einem Hub entlang der Strahleroberfläche diese gereinigt wird. Zu diesem Zweck ist ein in der Figur 1 nicht sichtbarer Antrieb, vorzugsweise pneumatisch oder hydraulisch, vorgesehen.

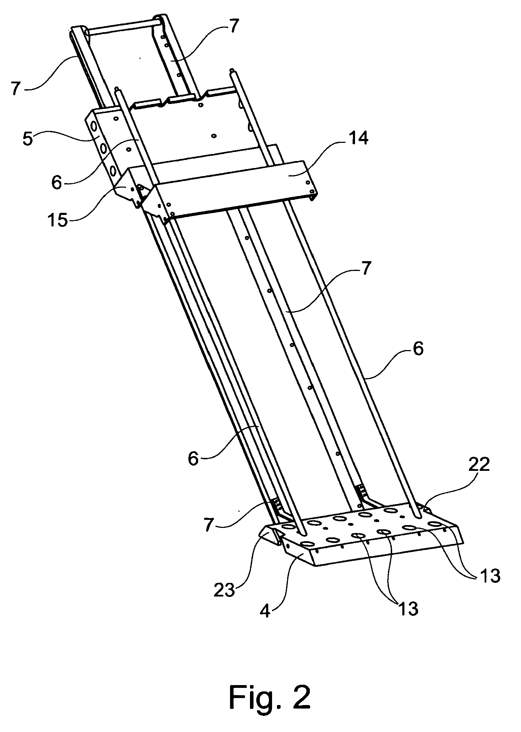

In der Figur 2 ist der mechanisch tragende Teil des Moduls 1 aus Figur 1 dargestellt, ohne die Strahler, die elektrischen Komponenten und den Sockel. Gleiche Bauelemente tragen gleiche Bezugsziffern. Es ist erkennbar, dass die Bodenplatte 4 eine Anzahl von zwölf Ausnehmungen 13 aufweist, in die die Strahler 2 mit ihrem unteren Ende, das dem elektrischen Anschluss abgewandt ist, eingesetzt werden. Die Strahler 2 werden dort gehalten. Der Rahmen 5 weist entsprechende Öffnungen für das obere Ende der Strahler 2 auf. Die Öffnungen sind in zwei stufenförmig gegeneinander versetzten Haltern 14 und 15 angeordnet. Die Öffnungen selbst sind in der Figur 2 nicht sichtbar. Die Figur 2 veranschaulicht, dass die Bodenplatte 4 im Betrieb horizontal in dem Kanal angeordnet ist und dass die Streben 6 und die Führungsschienen 7 in einem Winkel von etwa fünfzig gegen die Horizontale geneigt sind. Im selben Winkel nehmen auch die parallel zu den Streben 6 und den Führungsschienen 7 anzuordnenden Strahler im Betrieb ein. Es ist weiter dargestellt, dass die Führungsschienen 7 sich in Figur 2 hinter der Ebene befinden, die von den sechs benachbarten Strahlern gebildet wird.

Die in Figur 2 dargestellten Komponenten sind vorzugsweise aus korrosionsbeständigem Stahl gefertigt.

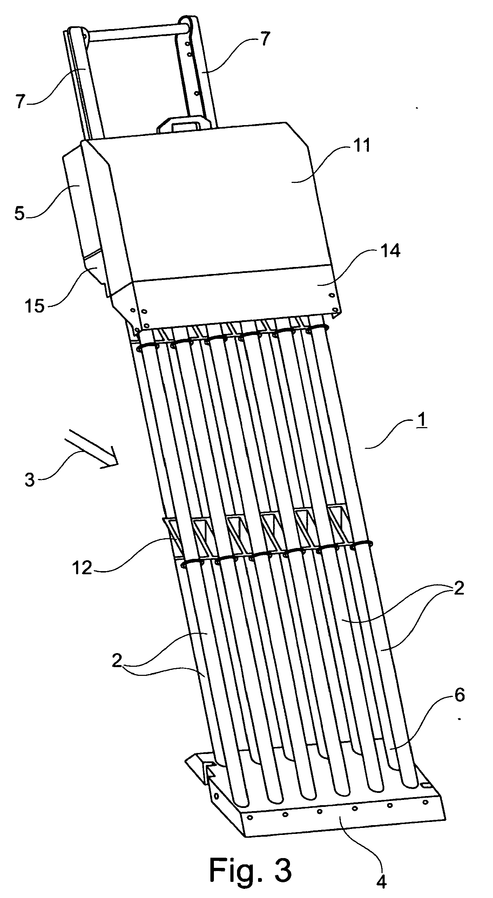

Die Figur 3 zeigt nochmals das Modul 1 mit den tragenden Elementen aus Figur 2 und den elektrischen Komponenten, die bereits in Figur 1 dargestellt waren, jedoch ohne den Figur 1 gezeigten Sockel. Gleiche Bauteile tragen wieder gleiche Bezugsziffern.

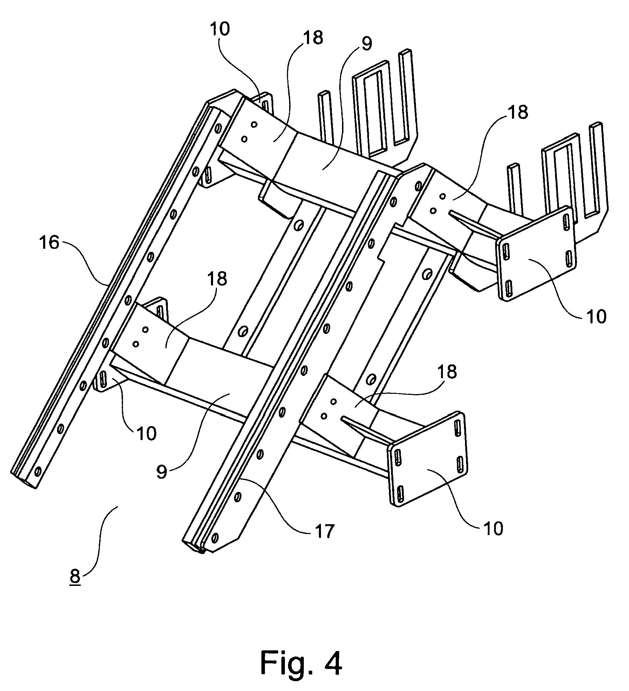

In der Figur 4 ist der Sockel 8 aus Figur 1 in einer perspektivischen Darstellung vergrößert dargestellt. Der Sockel 8 weist die Querstrebe 9 sowie die Befestigungsflansche 10 auf, die hier als Platten zur Befestigung an der Innenseite der Kanalwandung aufgebildet sind. Zwei Längsführungen 16 und 17 sind über Befestigungselemente 18 mit dem Querstreben 9 fest verbunden. Die Führungen 16 und 17 sind mit einem freien Querschnitt versehen, der in Längsrichtung der Führungen 16 und 17 verläuft und der so ausgebildet ist, dass die Führungsschienen 7 aus den Figuren 1 bis 3 längsverschieblich darin gelagert werden können. Vorzugsweise sind die Führungen 16 und 17 auch so ausgebildet, dass ein Verkippen der Führungsschienen 7 gegenüber den Führungen 16 und 17 nicht möglich ist. Bei dem Zusammenwirken der Führungsschienen 7 mit den Führungen 16 und 17 handelt es sich dann um eine Linearführung, deren Führungsrichtung im Wesentlichen parallel zu der Längsachse des Strahlers 2 orientiert ist.

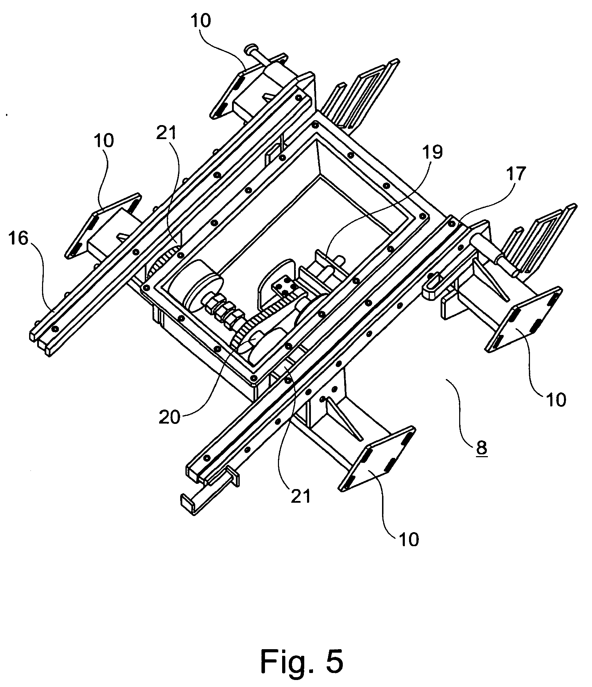

Die Figur 5 zeigt den Sockel 8 in einer ähnlichen Darstellung wie Figur 4, jedoch mit einem darin vorgesehenen Antrieb 19. Der Antrieb 19 ist hier als elektromotorischer Antrieb ausgebildet, der über ein vorzugsweise selbsthemmendes Getriebe 20 zwei Ritzel 21 antreibt. Die Ritzel 21 wiederum bewirken mit einer linearen Verzahnung nach Art einer Zahnstange zusammen, die an der in Figur 2 nicht sichtbaren Rückseite oder Unterseite der Führungsschienen 7 angeordnet ist.

Zusammen mit den Führungsschienen 7 ermöglicht der Sockel 8 mit dem Antrieb 19 dann ein gesteuertes, gradliniges Herausfahren des Moduls 1 aus dem Kanal durch in Betriebsetzung des Antriebs 19.

Im Betrieb kann das Modul gemäß Figur 1 in einer UV-Wasserbehandlungsanlage wie folgt eingesetzt werden. Es kann bei einer kleinen Anlage als einzelnes Modul in einem Kanal eingesetzt werden, der eine lichte Weite aufweist, die im Abstand von zwei sich gegenüberliegenden Befestigungsflanschen 10 entspricht. Die Höhe des Kanals von dem Boden bis zum Wasserspiegel entspricht dann etwa der senkrechten Projektion von der Unterseite der Bodenplatte 4 bis zu der Unterseite des Rahmens 5. In dem Kanal sind dann insgesamt zwölf Strahler 2 angeordnet, die zur Desinfektion des Wassers verwendet werden können. Der Kanal wird zweckmäßigerweise an seiner Oberseite abgedeckt, sodass keine UV-Strahlung austreten kann und dass weder Personen noch Gegenstände in den Kanal hineinfallen können.

Zur Wartung der Strahler 2 kann dann das Modul 1 durch in Betriebsetzung des Antriebs 19 nach oben über die Abdeckung des Kanals hinausgefahren werden. Der Antrieb 19 treibt dann über das Getriebe 20 die beiden Ritzel 21 an, die über ihren Eingriff mit der Verzahnung der Führungsschienen 7 dann die Führungsschienen 7 in den Führungen 16 und 17 entsprechend der Neigung der Führungsschienen 7 gegen die Horizontale schräg nach oben aus dem Kanal herausfahren. Dieser Vorgang wird vorzugsweise solange fortgesetzt bis die Bodenplatte 4 etwa bündig mit der Abdeckung des Kanals ist. Die Strahler 2 und auch die elektrischen Anschlüsse unterhalb der Abdeckung 11 sind dann frei zugänglich, ohne dass eine Öffnung in der Abdeckung des Kanals entsteht, die eine potentielle Gefahrenquelle darstellen würde. Die Bodenplatte 4 verdeckt den freien Querschnitt der Abdeckung des Kanals, in den das Modul eingefahren wird. Bei der dargestellten Ausführungsform ist besonders vorteilhaft, dass keine Führungen unterhalb der Wasserlinie erforderlich sind. Die Führungsschienen 7 werden ausschließlich in den Sockel 8 geführt, der sich vollständig oberhalb der Wasserlinie befindet. Diese Ausführung minimiert die erforderlichen Einbauten in dem Kanal und sorgt für eine besonders hohe Betriebssicherheit.

Bei größeren Kanälen können mehrere Module 1 in Strömungsrichtung 3 hintereinander angeordnet werden. Hierbei kann dann jedes Modul einzeln aus dem Kanal herausgefahren werden, wobei sich wieder keine große Öffnung in der Abdeckung des Kanals ergibt, da die Bodenplatte 4 des jeweiligen Moduls bei herausgefahrenem Modul die Oberseite so weit verschließt, dass die Gefahr des Hereinfallens von Personen oder Gegenständen in den Kanal nicht besteht.

Es können auch mehrere Module 1 nebeneinander quer zur Strömungsrichtung eingesetzt werden, wenn der Kanal eine mehrfache Breite des Abstandes zwischen zwei sich gegenüberliegenden Befestigungsflanschen 10 aufweist. Die randseitigen Module können dann jeweils mit zwei Befestigungsflanschen 10 an der Kanalwandung befestigt werden. Innerhalb des Kanals liegende Module werden mit den aufeinander zuweisenden Flanschen 10 aneinander befestigt und so in einer Richtung quer zu der Strömungsrichtung 3 miteinander verkettet. Die Funktion zum Herausfahren der Module aus dem Kanal für eine Wartung ist dabei dieselbe wie zuvor beschrieben. Jedes Modul kann einzeln durch in Betriebsetzung des Antriebs 19 schräg aus dem Kanal herausgefahren werden, ohne dass eine Lücke in der Abdeckung des Kanals entsteht. Das Modul steht dann über die Abdeckung nach oben hinaus und ist für Wartungszwecke einfach zugänglich. Diese Vorteile werden dadurch erzielt, dass das Modul 1 mit der Linearführung, die sich aus der Kombination der Führungsschienen 7 mit den Führungen 16 und 17 ergibt, in einer geraden Richtung aus dem Kanal heraus- und wieder hereingefahren werden kann.

Ein weiteres Merkmal ist aus der Zusammenschau von Fig. 1 und Fig. 2 ersichtlich. Die Bodenplatte 4 weist an einer in Strömungsrichtung 3 gesehen linken Schmalseite 22 etwa mittig einen Versatz auf, der quer zur Strömungsrichtung erfolgt. Der stromaufwärts liegende Abschnitt der Schmalseite 22 liegt um einen Betrag versetzt gegenüber dem stromabwärtigen Teil. Der Versatz entspricht etwa dem seitlichen Versatz der beiden Strahlerreihen der Strahler 2 gegeneinander. In der Figur 2 ist die Bodenplatte 4 von der anderen Seite aus sichtbar. Der Versatz ist hier in einer Schmalseite 23 entsprechend vorgesehen. Die Schmalseiten 22 und 23 verlaufen parallel zueinander.

Die Schmalseiten 22 und 23 sind insoweit komplementär zueinander, so dass benachbarte baugleiche Bodenplatten im wesentlichen lückenlos nebeneinander platziert werden können. Bei einer Anordnung mit mehreren Modulen nebeneinander kann dadurch erreicht werden, dass die Abstände und die relativen Positionen benachbarter Strahler 2 von zwei benachbarten Modulen in einer genau definiert sind, so dass sich keine Spalte mit geringer UV-Intensität zwischen den benachbarten Strahlern ergeben können. Gegebenenfalls können die Schmalseiten 22 und 23 auch noch mit komplementären Nuten und Stegen oder Zapfen versehen werden, die eine Sicherung zweier Bodenplatten 4 aneinander ermöglichen, so dass sie nicht parallel zu dem Boden des Kanals gegeneinander verschoben, wohl aber in Richtung der Führung voneinander getrennt werden können.

Der obere Rahmen 5 weist vorzugsweise den gleichen Versatz in seinen in Strömungsrichtung verlaufenden Seitenwänden auf wie die Bodenplatte 4.

## Patentansprüche

1. UV-Wasserbehandlungsanlage mit wenigstens einem Modul (1), welches eine Anzahl von langgestreckten UV-Strahlern (2) in einer Halterung enthält, wobei die Strahler (2) parallel zueinander ausgerichtet sind, **dadurch gekennzeichnet, dass** ein Sockel (8) mit wenigstens einer fest mit dem Sockel (8) verbundenen Führung (16, 17) und wenigstens eine mit der Halterung verbundene Führungsschiene (7) vorgesehen sind, wobei die Führungsschiene (7) in der Führung verschieblich gelagert ist.

2. UV-Wasserbehandlungsanlage nach Anspruch 1, **dadurch gekennzeichnet, dass** die Führung (16, 17) mit der Führungsschiene (7) eine Linearführung bildet.

3. UV-Wasserbehandlungsanlage nach Anspruch 2, **dadurch gekennzeichnet, dass** die Linearführung im Wesentlichen parallel zu der Längsachse der Strahler (2) verläuft.

4. UV-Wasserbehandlungsanlage nach einem der vorhergehenden Ansprüche, **dadurch gekennzeichnet, dass** ein Antriebsmittel (19, 20, 21) vorgesehen und eingerichtet ist, die Halterung mit den Strahlern (2) gegenüber dem Sockel (8) in Richtung der Führung (16, 17) zu verfahren.

5. UV-Wasserbehandlungsanlage nach einem der vorhergehenden Ansprüche, **dadurch gekennzeichnet, dass** das Modul (1) eine Bodenplatte (4) aufweist.

6. UV-Wasserbehandlungsanlage nach einem der vorhergehenden Ansprüche, **dadurch gekennzeichnet, dass** der Sockel mit Befestigungsvorrichtungen versehen ist, die beidseitig an sich gegenüberliegenden Seitenwandungen eines Kanals befestigbar sind.

7. UV-Wasserbehandlungsanlage nach einem der vorhergehenden Ansprüche, **dadurch gekennzeichnet, dass** der Sockel weist vorzugsweise wenigstens zwei Führungen aufweist, die seitlich angeordnet sind.

8. UV-Wasserbehandlungsanlage nach einem der vorhergehenden Ansprüche, **dadurch gekennzeichnet, dass** das Antriebsmittel ein Elektromotor ist, der über einen Ritzel eine mit dem Modul verbundene Zahnstange antreibt.

9. UV-Wasserbehandlungsanlage nach einem der vorhergehenden Ansprüche, **dadurch gekennzeichnet, dass** der Elektromotor über ein selbsthemmendes Getriebe mit dem Ritzel antriebsmäßig verbunden ist.

10. UV-Wasserbehandlungsanlage nach einem der vorhergehenden Ansprüche, **dadurch gekennzeichnet, dass** die Strahler (2) mit ihrer Längsachse parallel zu der Ebene der benachbarten Seitenwand des Kanals ausgerichtet und mit ihrer Längsachse in einem Winkel gegen die Strömungsrichtung geneigt sind.

11. UV-Wasserbehandlungsanlage nach einem der vorhergehenden Ansprüche, **dadurch gekennzeichnet, dass** der Winkel vorzugsweise zwischen dreißig und achtzig Grad und besonders bevorzugt zwischen vierzig und sechzig Grad zwischen der Strahlerlängsachse und der im Wesentlichen horizontalen Strömungsrichtung beträgt.

## Claims

1. UV water treatment plant having at least one module (1) which contains a number of elongate UV emitters (2) in a frame, the emitters (2) being aligned parallel to one another **characterised in that** a base (8) having at least one guide (16, 17) connected firmly to the base (8) and at least one guide rail (7) connected to the frame are provided, the guide rail (7) being mounted displaceably in the guide.

2. UV water treatment plant according to Claim 1, **characterised in that** the guide (16, 17) forms a linear guide with the guide rail (7).

3. UV water treatment plant according to Claim 2, **characterised in that** the linear guide extends essentially parallel to the longitudinal axis of the emitters (2).

4. UV water treatment plant according to one of the preceding claims, **characterised in that** a drive means (19, 20, 21) is provided and is configured in order to move the frame with the emitters (2) relative to base (8) in the direction of the guide (16, 17).

5. UV water treatment plant according to one of the preceding claims, **characterised in that** the module has a base plate (4).

6. UV water treatment plant according to one of the preceding claims, **characterised in that** the base is provided with fastening means which can be fastened on both sides to mutually opposing side walls of a channel.

7. UV water treatment plant according to one of the preceding claims, **characterised in that** the base preferably has at least two guides, which are arranged laterally.

8. UV water treatment plant according to one of the preceding claims, **characterised in that** the drive means is an electric motor which drives a rack connected to the module by means of a pinion.

9. UV water treatment plant according to one of the preceding claims, **characterised in that** the electric motor is drivingly connected to the pinion by means of a self-locking gear.

10. UV water treatment plant according to one of the preceding claims, **characterised in that** the emitters (2) are aligned with their longitudinal axis parallel to the plane of the neighbouring side wall of the channel and are inclined with their longitudinal axis at an angle with respect to the flow direction.

11. UV water treatment plant according to one of the preceding claims, **characterised in that** the angle is preferably between thirty and eighty degrees, and particularly preferably between forty and sixty degrees, between the emitter longitudinal axis and the essentially horizontal flow direction.

## Revendications

1. Installation de traitement de l'eau par UV comprenant au moins un module (1) qui contient un certain nombre d'émetteurs de rayonnement UV (2) allongés, disposés dans un support, ces émetteurs de rayonnement (2) étant parallèles les uns aux autres, **caractérisée en ce qu'**un socle (8) comportant au moins une glissière (16, 17) reliée de manière fixe au socle (8) et au moins un rail de guidage (7) relié au support sont prévus, le rail de guidage (7) étant logé mobile dans la glissière.

2. Installation de traitement de l'eau par UV selon la revendication 1, **caractérisée en ce que** la glissière (16, 17) forme avec le rail de guidage (7) un guidage linéaire.

3. Installation de traitement de l'eau par UV selon la revendication 2, **caractérisée en ce que** le guidage linéaire s'étend essentiellement parallèlement à l'axe longitudinal des émetteurs de rayonnement (2).

4. Installation de traitement de l'eau par UV selon une des revendications précédentes, **caractérisée en ce qu'**un moyen d'entraînement (19, 20, 21) est prévu et installé de sorte à déplacer le support avec les émetteurs de rayonnement (2) par rapport au socle (8) en direction de la glissière (16, 17).

5. Installation de traitement de l'eau par UV selon une des revendications précédentes, **caractérisée en ce que** le module (1) présente une plaque de fond (4).

6. Installation de traitement de l'eau par UV selon une des revendications précédentes, **caractérisée en ce que** le socle est pourvu de dispositifs de fixation qui sont susceptibles d'être rattachés latéralement sur les parois opposées d'un canal.

7. Installation de traitement de l'eau par UV selon une des revendications précédentes, **caractérisée en ce que** le socle présente de préférence au moins deux glissières qui sont agencées latéralement.

8. Installation de traitement de l'eau par UV selon une des revendications précédentes, **caractérisée en ce que** le moyen d'entraînement est un moteur électrique qui entraîne à l'aide d'un pignon une crémaillère reliée au module.

9. Installation de traitement de l'eau par UV selon une des revendications précédentes, **caractérisée en ce que** le moteur électrique est relié en entraînement au pignon à l'aide d'un mécanisme d'entraînement à blocage automatique.

10. Installation de traitement de l'eau par UV selon une des revendications précédentes, **caractérisée en ce que** les émetteurs de rayonnement (2) sont alignés avec leur axe longitudinal parallèlement au plan de la paroi latérale adjacente du canal et sont inclinés en formant en angle avec leur axe longitudinal par rapport à la direction d'écoulement.

11. Installation de traitement de l'eau par UV selon une des revendications précédentes, **caractérisée en ce que** l'angle se situe de préférence entre trente et quatre-vingt degrés et, de façon particulièrement préférée, entre quarante et soixante degrés entre l'axe longitudinal des émetteurs de rayonnement et la direction d'écoulement essentiellement horizontale.
